# EUROPEAN PATENT APPLICATION

(11) **EP 4 485 477 A1**
(43) Date of publication of application: **01.01.2025**
(21) Application number: 23182268.5
(22) Date of filing: 29.06.2023
(51) Int. Cl.: G16H 50/30, G16H 50/20, G16H 50/70, G16H 10/60

(54) **PREDICTIVE ASSESSMENT OF HEALTH RISK PARAMETERS REGARDING GESTATIONAL DIABETES MELLITUS AND PREECLAMPSIA**

(71) Applicant: Koninklijke Philips N.V., 5656 AG Eindhoven (NL)
(72) Inventor: ASVADI, Sima, Eindhoven (NL); JOHNSON, Mark Thomas, Eindhoven (NL); PALERO, Jonathan Alambra, Eindhoven (NL); WU, Yanqi, Eindhoven (NL); LONG, Xi, Eindhoven (NL)
(74) Representative: Philips Intellectual Property & Standards

(57) **Abstract**

The invention provides a computer-implemented method for predictive assessment of a health risk parameter, such as a risk for diabetes, in particular gestational diabetes mellitus, and/or pre-eclampsia, for a woman based on at least one pregnancy-related parameter, comprising the following steps: providing an assessment of the health risk parameter using a first machine-learning model (1) if the pregnancy-related parameter fulfils a first condition; providing an assessment of the health risk parameter using a second machine-learning model (2) if the pregnancy-related parameter fulfils a second condition; and making available the health risk parameter.

## Description

### FIELD OF THE INVENTION

The present invention generally concerns the field of health risk prediction, in particular in the context of coming and/or ongoing pregnancies. Exemplary embodiments of the present invention have applications with respect to gestational diabetes mellitus (GDM) or preeclampsia. High accuracy in prediction can be accomplished using only limited data and limited computational resources.

### BACKGROUND OF THE INVENTION

Digitalization is ubiquitous in today's world and fundamentally changes the way we live and communicate. It may bring many unprecedented advantages to society. To that end, digitalization not only enables a more efficient realization of traditional processes, but also allows very different and new approaches to problems that were not available before. This is equally true for the health sector.

Gestational diabetes mellitus (GDM) is a type of diabetes that can develop during pregnancy. Around 1 in 7 pregnant women will develop GDM during pregnancy. GDM develops if excessive glucose is accumulated in maternal blood. During pregnancy certain pregnancy hormones secreted by the placenta, such as oestrogen, cortisol and lactogen inhibit insulin functioning. Insulin therefore becomes less effective in transferring glucose from the blood stream to the expecting mother's tissues. This process is essential to ensure an adequate nutrient supply to the baby. However, the process also causes insulin resistance in the mother's body, which also increases as the pregnancy advances. If the mother's body does not secrete enough insulin to balance this resistance, her blood sugar increases and GDM occurs.

Women with gestational diabetes do not have diabetes before their pregnancy and their GDM usually resolves after giving birth. Gestational diabetes can occur at any stage of a pregnancy but is often diagnosed during the second or third trimester. Women who develop GDM might need to take medication to regulate their blood glucose. If the level of blood glucose is not controlled, it might contribute to developing other pregnancy related complications such as pre-eclampsia (high blood pressure and protein in the urine) and in some cases might require a caesarean section. Furthermore, they might become prone to developing diabetes later in life.

For babies, potential consequences of uncontrolled gestational diabetes of the mother include growing larger than they normally would or having hypoglycaemia (low blood sugar) after birth. Generally, starting pregnancy with a (significantly) high weight (i.e., BMI), having diabetes in the family, or having developed GDM in a previous pregnancy might increase the chance of GDM for the current pregnancy.

Scientific research has shown that early lifestyle modifications during pregnancy can reduce the chance of developing GDM. This effect is enhanced if lifestyle adjustments are made early on, typically before week 15 of pregnancy, and maintained during pregnancy.

There is thus a need to accurately predict the risk of GDM at an early stage during pregnancy.

The accuracy of models for early risk prediction of GDM may depend on the risk factors that are included in training the model. It is known from the prior art to use factors in GDM risk prediction models such as: Age, weight or BMI, Ethnicity(race), family history of diabetes, parity, gravidity and history of GDM, as well as certain blood-based biomarkers such as hsCRP, SHBG, adipokines as well as inflammatory markers like TNF-alpha. The present GDM early risk prediction models are, however, providing limited risk prediction accuracy with area under curve (AUC) in the range between 0.70 to 0.80. This has hence disadvantages resulting from the limited accuracy of the prediction, leading to false decisions in treatment, preventions and adaptions of lifestyle. Higher accuracy and precision would hence be highly desirable.

It is hence an object of the present invention to provide methods for improving the risk prediction accuracy of risk prediction models, such as particularly GDM early risk prediction models.

Often, data for model training is difficult to obtain and quantitatively limited. It is hence another object of the invention to provide for high-precision results based on limited data and limited computational resources.

### SUMMARY OF THE INVENTION

A solution to the problem has now been devised by the subject-matter of the independent claims. Accordingly, a computer-implemented method for predictive assessment of a health risk parameter is provided as defined in independent claim 1. The health risk parameter may, for example, be a risk for diabetes, in particular gestational diabetes mellitus, and/or pre-eclampsia. The health risk parameter may be defined for a woman based on at least one pregnancy-related parameter. The method may comprise providing an assessment of the health risk parameter using a first machine-learning model if the pregnancy-related parameter fulfils a first condition. The method may comprise providing an assessment of the health risk parameter using a second machine-learning model if the pregnancy-related parameter fulfils a second condition. The health risk parameter may then be made available, e.g., so that actions can be taken and/or subsequent analyses can be performed.

In an example, the pregnancy-related parameter comprises a number of pregnancies already carried out by the woman. For example, if the woman has her first pregnancy, the first machine-learning model may be used for prediction. If the woman is having her second (or also: subsequent, that is, second or subsequent) pregnancy, the second machine-learning model may be used for prediction.

In this way, by relying on the two models, the risks for both first and second pregnancy can be correctly assessed. This assessment that uses separately trained models requires less processing power, already in the assessment, but particularly also in the training of the models, among other things because smaller datasets can be used to train the model to achieve good accuracy. This is furthermore of even higher relevance than one might naively assume, since in many cases access and availability of large datasets are highly limited. Accordingly, enabling the use of smaller training datasets not only makes the disclosed methods feasible in the first place but also helps achieve the following technical advantages:
1. Faster training: Smaller datasets may require less computational resources and time to train the models, as compared to larger datasets. Training a model on a smaller dataset can be beneficial when there are time or resource constraints.
2. Reduced overfitting: Overfitting may occurs when a model learns to perform well on the training data but fails to generalize to unseen data. With smaller datasets, the models have fewer opportunities to memorize specific examples and are encouraged to learn more general patterns, thus reducing overfitting.
3. Simplified data management: Smaller datasets may be easier to manage and/or store. They require less storage space and can be processed more quickly, making it more convenient to work with limited resources.
4. Improved model interpretability: Smaller datasets can lead to simpler models with fewer parameters. Such models are often easier to interpret and understand. Interpretability is valuable for gaining insights into how the models make predictions and for building trust in the models' decision-making process.
5. Resource efficiency: Training the models on smaller datasets requires fewer computational resources, including memory, processing power, and energy consumption. This advantage is particularly beneficial in resource-constrained environments or when deploying models on edge devices with limited capabilities.

In developing the invention, the inventors have among other things realized that using single models in the prediction of risk of developing GDM may result in over-confidence in the prediction in first pregnancy. However, after thorough analysis, data shows that the AUC is actually lower for the model deployed on the first pregnancy cohort than for a single cohort. Furthermore, single models in the prediction of the risk assessment of developing GDM in a second pregnancy will be given with over-caution wherein thoroughly analyzed data shows that the AUC is actually higher for a model deployed on a second pregnancy cohort than for a single cohort. In this way, the invention provides prediction results of higher quality, with less resources. Less data and less training are needed which enables high-quality prediction also in low resource computational environments, such as local risk assessment (personal/professional) devices.

Different types of conditions may be suitable for use within the present invention. In one example embodiment, the first condition may be that the woman has a first-time pregnancy (nulliparous). The second condition may be that the women has a second pregnancy or that the woman has a pregnancy subsequent to the first one (multiparous).

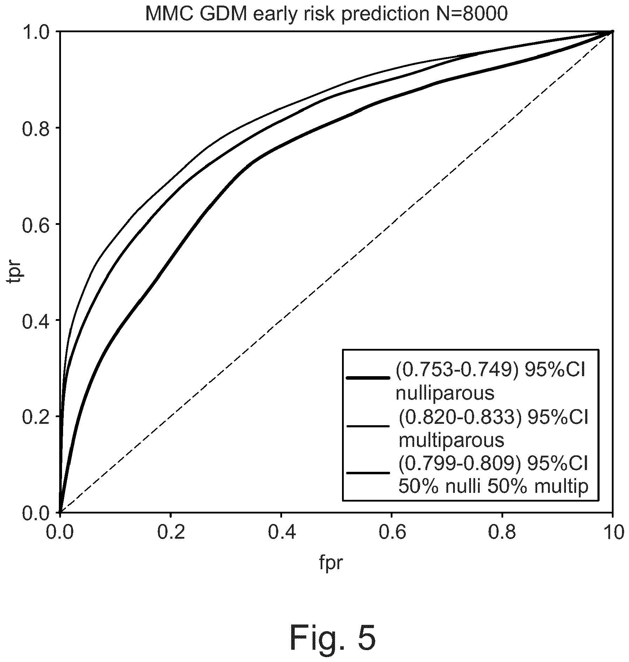

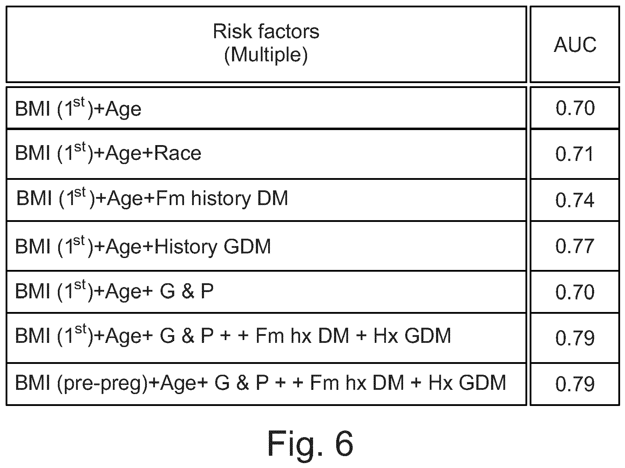

Experiments have shown that AUC achieved using the aspects of the present invention significantly better matches the real risk, as compared to single model approaches (see, e.g., Fig. 6 and the respective figure description further below). As can be seen in detail in Fig. 5, the effect of the previous history causes a change in prediction accuracy for 1st time mothers (nulliparous) with AUC = 0.735 to 0.749 (95%CI) and multiple delivery mothers (multiparous), with AUC = 0.820 to 0.833 (95%CI).

Conditions may comprise further subsequent pregnancies, e.g., separate models for third or fourth pregnancy could be envisaged. In a preferred embodiment, separate models may also be trained on separate cohorts. As the data shows, this may entail additional beneficial effects.

Alternatively, they can be trained a combined cohort and, optionally, may later be deployed on separate cohorts. In an exemplary embodiment of the present invention, the effect of the previous history may cause in this case a change in prediction accuracy for 1st time mothers (nulliparous) with AUC = 0.743 to 0.763 (95%CI) and multiple delivery mothers (multiparous) with AUC = 0.814 to 0.826 (95%CI). The accuracy of prediction in this alternative embodiment using the combined cohort for training is hence lower than the accuracy using a training with the individual cohorts (of the same size, to assure a reasonable comparability). Putting it differently, a contrario, it can be deduced from the data that a separate cohort training has additional beneficial effects for the prediction accuracy that is achieved.

The medical history of the pregnant woman may also play a crucial role. This is particularly the case for the medical history with respect to the risk to be assessed. This could hence be, e.g., a GDM history for a second or subsequent pregnancy. This may be used as a third condition in the machine-learning topology. In an example, a second model is only used if the pregnant woman has had a GDM history. Otherwise, a third model may be employed. Ideally, also these models are trained and deployed on separate cohorts, but also here joint cohort training is possible as an alternative.

The resulting three model topology could be shown to yield better results. It is clear to the skilled person that this can be further generalized for several (any number of) pregnancies and/or models.

The medical history of the pregnant woman may also play a crucial role with respect to other diseases than a disease with respect to which the risk is to be assessed. For example, for GDM risk assessment, other factors such as histories of pre-eclampsia, hypertension and their parity-specific occurrence may be taken into account.

The invention facilitates the creation of preventive intervention routines (such as, e.g., lifestyle modifications or preventive medicine) for those women who have been identified to have a high risk for a specific disease during the course of their pregnancy, such as GDM or pre-eclampsia.

In an embodiment, the invention may be practiced with respect to hypertensive disorders of pregnancy. Hypertensive disorders of pregnancy are the most common medical complications in pregnancy, affecting up to 10% of all pregnancies. Hypertensive disorders of pregnancy include gestational hypertension, white-coat hypertension, masked hypertension, chronic hypertension, chronic hypertension with superimposed pre-eclampsia or PE, PE, eclampsia, hemolysis, Elevated Liver enzymes and Low Platelets (HELLP) syndrome and postpartum hypertension. These hypertension disorders can contribute to health problems during pregnancy, such as intrauterine growth restriction, placental abruption, preterm birth and caesarean birth. PE affects 2% to 8% of pregnancies and is a leading cause of maternal morbidity and mortality worldwide (>63,000 cases of maternal deaths worldwide reported annually). The rates of PE in the United States have been steadily rising being a leading cause of maternal and fetal/neonatal morbidity and mortality.

Early identification of women at higher risk for preeclampsia may aid early prevention and treatment. Although a plethora of preeclampsia prediction models have been developed in recent years, individualised prediction of preeclampsia is rarely used in clinical practice. It is therefore beneficial to apply the present invention also to preeclampsia (as well as other mentioned diseases).

The present invention also provides a computer program, or a computer-readable medium storing a computer program. The computer program may comprise instructions which, when the program is executed on a computer and/or a computer network, cause the computer and/or the computer network to carry out any of the methods disclosed herein.

The present invention also provides a machine-learning model data structure. The data structure may embody a machine-learning model, in particular a machine- learning model, for predictive assessment of a health risk parameter. The model may be trained, or additionally prepared, using any of the methods disclosed herein.

### BRIEF DESCRIPTION OF THE DRAWINGS

The disclosure may be better understood by reference to the following drawings:
Fig. 1 A flow diagram illustrating several steps in a possible embodiment of the present invention for predictive assessment of health risk parameters,
Fig. 2 Plots illustrating a predicted risk over time using several separate machine-learning models for risk prediction in a possible embodiment of the present invention,
Fig. 3 A flow diagram illustrating several steps in a possible embodiment of the present invention for preparing, training and deploying models for predictive assessment of health risk parameters,
Fig. 4a A diagram illustrating training and testing of machine-learning models according to a preferred embodiment of the present invention,
Fig. 4b A diagram illustrating training and testing of machine-learning models according to an alternative embodiment of the present invention,
Fig. 5 Several plots illustrating results achieved by an embodiment of the present invention in terms of receiver operating characteristics and corresponding AUC values,
Fig. 6 A table that shows AUC performance results from single models (as known from the prior art) when supplementary medical risk factors are taken into account.

### DETAILED DESCRIPTION OF THE INVENTION

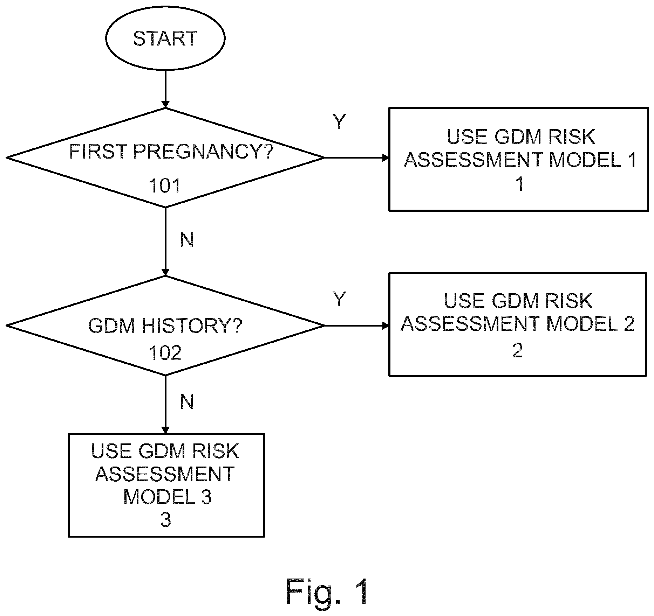

Fig. 1 shows a flow diagram illustrating several steps in a possible embodiment of the present invention for predictive assessment of health risk parameters.

Using a method for predictive health risk assessment as provided for by embodiments of the present invention, it is first determined at step 101 if a current or ongoing pregnancy is a first pregnancy of the women for who the health risk is examined. If the answer to this question is YES (example of first condition in this embodiment), a first machine-learning model 1 will be relied upon for the assessment. If the answer to this question is NO (example of second condition in this embodiment), a second machine-learning model 2 may be relied upon for the assessment. Alternatively and/or supplementarily, if the answer to this question is NO, additional factors may be taken into account. These can lead to further splitting into submodels. For example, at step 102 it may be determined if a woman (already) has a history with respect to the risk/disease, e.g., has a GDM history. Depending on the answer, the health risk (GDM risk) prediction task may be deferred to the second machine-learning model 2 or a third machine-learning model 3, respectively. The advantages of these splittings on precision and computational effort and resources as well as reduced required amounts of training data are described in more detail in the summary section.

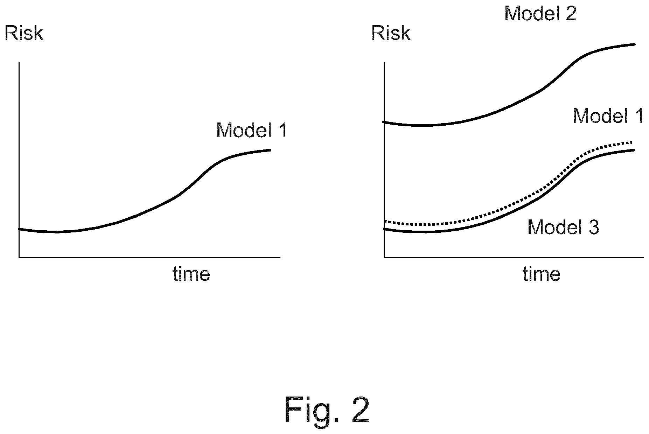

Fig. 2 shows several plots illustrating a predicted risk over time using several separate machine-learning models, i.e., the first model 1, second model 2 and third model 3 explained above, for risk prediction in a possible embodiment of the present invention.

The curves have been generated using the models 1, 2 and 3 after having been trained according to an embodiment of the present invention. In the left part of Fig. 2, a GDM risk curve is shown (plotted over time passed) for the first model 1, e.g., for a first-time pregnancy. The right part of Fig. 2 shows corresponding curves for exemplary models 2 and 3, here for subsequent (i.e., second and subsequent) pregnancies. The dashed line is a reproduction of model 1 and is shown for comparison purpose only. The data indicates that the chance of developing GDM during a second or subsequent pregnancy is around an order of magnitude higher for women with a GDM history.

The models may accept any sort of input particularly with respect to additional medical parameters of the mother-to-be. They may provide personalized risk-level assessment, personalized feedback, recommendations as well as preventive paths.

Qualitatively similar results have been obtained by the present inventors for the case of preeclampsia, using the similar techniques.

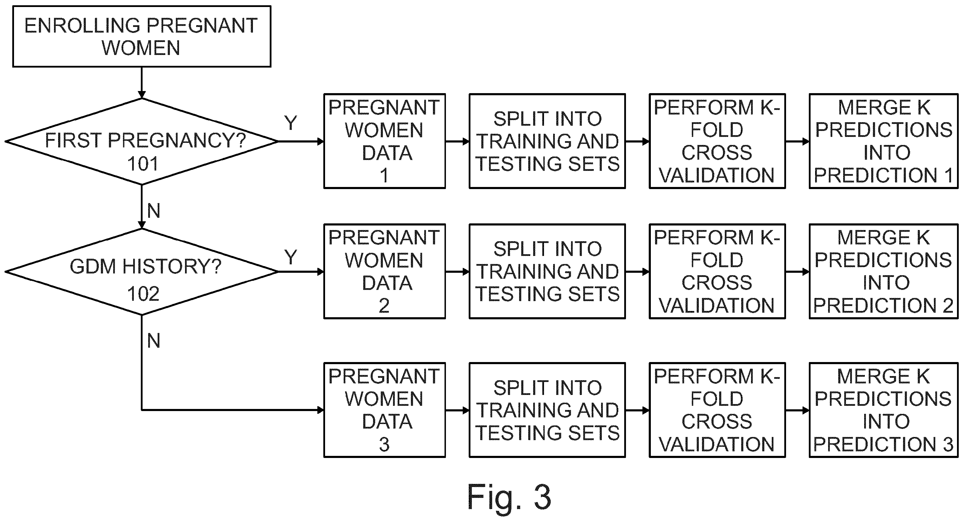

Fig. 3 shows a flow diagram illustrating several steps in a possible embodiment of the present invention for preparing, training and deploying models for predictive assessment of health risk parameters.

The exemplary machine-learning model structure and method shown in Fig. 3 may be, according to an embodiment, prepared and trained as follows. Collection of data may comprise enrolling, collecting and screening data from pregnant women including age, BMI or gestational weight or GWG classification ethnicity (race), family history of diabetes, parity, gravidity, history of GDM, and more. A separation of data into several, here three, data sets may be performed: 1) first pregnancy; 2) subsequent pregnancy with GDM history, and; 3) subsequent pregnancy with no GDM history. Then, the data may be split, preferably randomly, into a training set (e.g. 80%) and a testing set (e.g. 20%), for each data set.

A k-fold cross validation may be performed, e.g., k = 5, wherein for each fold, the GDM model is trained using the risk factors and labels from the collected data by supervised machine learning using decision tree ensemble learning algorithms, e.g., Extreme Gradient Boosting or XGBoost. Lastly, one may merge the prediction from the k-folds into prediction results for each data set.

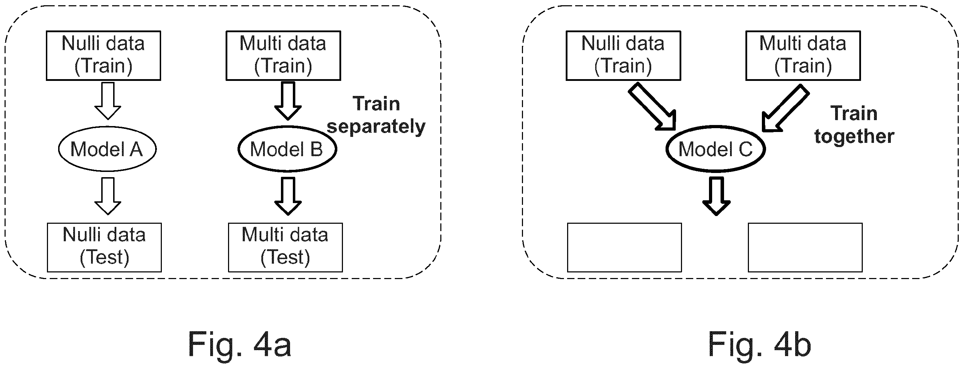

Figs. 4a, 4b show diagrams illustrating training and testing of machine-learning models according to possible embodiments of the present invention. Therein, Fig. 4a shows a diagram illustrating training and testing of machine-learning models according to a preferred embodiment of the present invention.

As can be seen in Fig. 4a, two exemplary models, here denoted for the sake of generality A and B, can be trained separately and be deployed on separate cohorts. Models A and B shown here could be the first 1 and second machine-learning model 2, but the concepts illustrated here could equally well be applied to e.g. second and third (or subsequent) machine-learning models. As has been elaborated in the summary section, this training and deployment strategy may yield particularly good results.

Fig. 4b shows a diagram illustrating training and testing of machine-learning models according to an alternative embodiment of the present invention. As can be seen in Fig. 4b, two exemplary models, here denoted for the sake of generality A and B, can be trained together and be deployed on separate cohorts. Models A and B shown here could be the first 1 and second machine-learning model 2, but the concepts illustrated here could equally well be applied to e.g. second and third (or subsequent) machine-learning models. As has been elaborated in the summary section, this strategy may not yield the same quality of results that can be achieved with the embodiment depicted in Fig. 4a. However, it may provide a good alternative. Compared to the prior art, in particular the single-model-only topology, much better results can still be expected. Further details and quantitative support can be found in the summary section. Embodiments of the invention can hence be practiced by splitting the cohort of the dataset to train and/or by deploying not one, but two (or more) risk models. In a preferred embodiment, the invention may be practiced by splitting the cohort of the dataset to train and by deploying two (or more) (corresponding) risk models.

Fig. 5 shows several plots illustrating results achieved by embodiments of the present invention in terms of receiver operating characteristics and corresponding AUC values. The predictions are shown for a fixed size of cohorts N=8000 (to ensure a fair comparison, as accuracy generally slightly increases as the number N of cases increases). The lowest of the three curves (excluding the dashed line) is based on the nulliparous dataset (first time pregnancies). The highest of the three curves is based on the multiparous dataset. The curve in between is based on a 50% : 50% mixture. As can be seen, the AUC for first time pregnancies is actually lower. Embodiments of the invention thereby avoid having a (wrong) predicted over-confidence for the first time pregnancies. As can be seen as well, the AUC for multiparous is actually higher. Embodiments of the invention thereby avoid having a (wrong) predicted over-caution for the multiparous cases. Due to this significant increase in prediction, it becomes possible to get reliable results already from processing small datasets. This avoids collection of the otherwise very large datasets which is very expensive as well as their data processing and model training that consumes, often in computing clouds, big amounts of space and computational power. Therefore, using the invention, more accurate predictions can be provided to users with less efforts already in less powerful computational environments.

Fig. 6 shows a table that shows AUC performance results from single models (as known from the prior art) when supplementary medical risk factors are taken into account.

As more and more parameters (such as, direct or indirect, risk factors) are added, accuracy of the model in consideration generally increases. In the best case, taking into account several factors in a complex single model, a result of .79 AUC could be obtained. The results, here only shown as examples, indicate a saturation effect with respect to AUC achieved.

As has been described in more detail above, AUC values higher than .79 AUC can be obtained using the present invention. The evidence hence supports that results of higher quality can be obtained by using the present invention as compared to the single universal model topology known from the prior art. It seems furthermore highly plausible, in view of the results studied, that the new results obtained may hardly ever be obtained using a single universal model, and particularly not using the same size of data samples and computational power.

That said, the use of embodiments of the invention may additionally benefit from using a vast variety of relevant risk factor parameters in the context of the machine-learning models. In one example, different sets of parameters can be used in the different submodels, e.g., in the first 1, second 2 (and potentially third 3) machine-learning models. In this way, an optimized balance between quality of result and computational effort can be found. Also, different abundances/availabilities of relevant data in the respective categories can duly be taken into consideration.

Qualitatively similar results have been obtained by the present inventors for the case of preeclampsia, using the same technique.

The embodiment(s) discussed in the figure descriptions may be combined with any of the preferred additional features for the invention disclosed herein.

Although some aspects have been described in the context of a device, it is clear that these aspects also represent a description of the corresponding process, where a block or device corresponds to a process step or a function of a process step. Similarly, aspects described in the context of a process step also constitute a description of a corresponding block or element or feature of a corresponding device. Also, features disclosed in the context of a certain process, device or model may be used in a corresponding other process, device or model. For example, features of the encoder model may be used analogously in a corresponding decoder model, and vice versa.

Embodiments of the invention may be implemented in a computer system. The computer system may be a local computing device (e.g., personal computer, laptop, tablet computer, or cell phone) having one or more processors and one or more storage devices, or may be a distributed computing system (e.g., a cloud computing system having one or more processors or one or more storage devices distributed at various locations, for example, at a local client and/or one or more remote server farms and/or data centers). The computer system may comprise any circuitry or combination of circuitry. In one embodiment, the computer system may comprise one or more processors, which may be of any type. As used herein, processor may mean any type of computing circuit, such as, but not limited to, a microprocessor, a microcontroller, a complex instruction set microprocessor (CISC), a reduced instruction set microprocessor (RISC), a very long instruction word (VLIW; VLIW) microprocessor, a graphics processor, a digital signal processor (DSP), a multi-core processor, a field-programmable gate array (FPGA), or any other type of processor or processing circuit. Other types of circuitry that may be included in the computer system may include a custom-built circuit, an application-specific integrated circuit (ASIC), or the like, such as one or more circuits (e.g., a communications circuit) for use in wireless devices such as cellular phones, tablet computers, laptop computers, two-way radios, and similar electronic systems. The computer system may include one or more storage devices, which may include one or more storage elements suitable for the particular application, such as main memory in the form of random access memory (RAM), one or more hard drives, and/or one or more drives that handle removable media, such as CDs, flash memory cards, DVDs, and the like. The computer system may also include a display device, one or more speakers, and a keyboard and/or controller, which may include a mouse, trackball, touch screen, voice recognition device, or any other device that allows a system user to input information to and receive information from the computer system.

Some or all of the method steps may be performed by (or using) a hardware device, such as may be a processor, a microprocessor, a programmable computer, or an electronic circuit. In some embodiments, one or more of the key method steps may be performed by such a device.

Depending on certain implementation requirements, embodiments of the invention may be implemented in hardware or software. The implementation may be performed using a non-volatile storage medium such as a digital storage medium, such as a floppy disk, a DVD, a Blu-Ray, a CD, a ROM, a PROM and EPROM, an EEPROM, or a FLASH memory, on which electronically readable control signals are stored that interact (or are capable of interacting) with a programmable computer system such that the respective process is performed. Therefore, the digital storage medium may be computer readable.

Some embodiments according to the invention include a data carrier having electronically readable control signals that can interact with a programmable computer system so as to perform any of the methods described herein.

In general, embodiments of the present invention may be implemented as a computer program product having program code, the program code being effective to perform any of the methods when the computer program product is running on a computer. For example, the program code may be stored on a machine-readable medium.

Further embodiments include the computer program for performing any of the methods described herein stored on a machine-readable medium.

In other words, an example embodiment of the present invention therefore includes a computer program having program code for performing any of the methods described herein when the computer program is running on a computer.

Thus, another embodiment of the present invention is a storage medium (or a digital storage medium or a computer-readable medium) comprising a computer program stored thereon for performing any of the methods described herein when executed by a processor. The data carrier, digital storage medium, or recorded medium is generally tangible and/or non-transitory. Another embodiment of the present invention is an apparatus as described herein comprising a processor and the storage medium.

Thus, another embodiment of the invention is a data stream or signal sequence representing the computer program for performing any of the methods described herein. For example, the data stream or signal sequence may be configured to be transmitted over a data communication link, such as over the Internet.

Another example embodiment comprises a processing means, for example, a computer or programmable logic device, configured or adapted to perform any of the methods described herein.

A further example embodiment comprises a computer having installed thereon the computer program for performing any of the methods described herein.

Another embodiment according to the invention comprises a device or system configured to transmit (for example, electronically or optically) a computer program for performing any of the methods described herein to a receiver. The receiver may be, for example, a computer, a mobile device, a storage device, or the like. The device or system may include, for example, a file server for transmitting the computer program to the receiver.

In some embodiments, a programmable logic device (e.g., a field programmable gate array, FPGA) may be used to perform some or all of the functionalities of the methods described herein. In some embodiments, a field programmable gate array may cooperate with a microprocessor to perform any of the procedures described herein. In general, the methods are preferably performed by any hardware device.

Embodiments may be based on using an artificial intelligence, particularly a machine learning model or machine learning algorithm. Machine learning may refer to algorithms and statistical models that computer systems can use to perform a particular task without using explicit instructions, rather than relying on models and inference. For example, machine learning may use, instead of a rule-based transformation of data, a transformation of data that can be inferred from an analysis of history and/or training data. For example, the content of images may be analyzed using a machine learning model or using a machine learning algorithm. In order for the machine learning model to analyze the content of an image, the machine learning model may be trained using training images as input and training content information as output. By training the machine learning model with a large number of training images and/or training sequences (e.g., words or sentences) and associated training content information (e.g., labels or annotations), the machine learning model "learns" to recognize the content of the images so that the content of images not included in the training data can be recognized using the machine learning model. The same principle can be used for other types of sensor data as well: By training a machine learning model using training sensor data and a desired output, the machine learning model "learns" a transformation between the sensor data and the output, which can be used to provide an output based on non-training sensor data provided to the machine learning model. The provided data (e.g., sensor data, metadata, and/or image data) may be preprocessed to obtain a feature vector that is used as input to the machine learning model.

Machine learning models can be trained using training input data. The above examples use a training method called supervised learning. In supervised learning, the machine learning model is trained using a plurality of training samples, where each sample may include a plurality of input data values and a plurality of desired output values, i.e., each training sample is associated with a desired output value. By specifying both training samples and desired output values, the machine learning model "learns" which output value to provide based on an input sample that is similar to the samples provided during training. In addition to supervised learning, semi-supervised learning can also be used. In Semi-Supervised Learning, some of the training samples lack a desired output value. Supervised learning can be based on a supervised learning algorithm (e.g., a classification algorithm, a regression algorithm, or a similarity learning algorithm). Classification algorithms can be used when the outputs are restricted to a limited set of values (categorical variables), i.e., the input is classified as one of the limited set of values. Regression algorithms can be used when the outputs exhibit some numerical value (within a range). Similarity learning algorithms can be similar to both classification and regression algorithms, but are based on learning from examples using a similarity function that measures how similar or related two objects are. In addition to supervised learning or semi-supervised learning, unsupervised learning can be used to train the machine learning model. In Unsupervised Learning, (only) input data may be provided and an Unsupervised Learning algorithm may be used to find structure in the input data (e.g., by grouping or clustering the input data, finding commonalities in the data). Clustering is the assignment of input data comprising a plurality of input values into subsets (clusters) such that input values within the same cluster are similar according to one or more (predefined) similarity criteria, while they are dissimilar to input values comprised in other clusters.

Reinforcement learning is a third group of machine learning algorithms. In other words, reinforcement learning can be used to train the machine learning model. In reinforcement learning, one or more software actors (called "software agents") are trained to take actions in an environment. Based on the actions taken, a reward is calculated. Reinforcement learning is based on training the one or more software agents to select actions such that the cumulative reward is increased, resulting in software agents that become better at the task they are given (as evidenced by increasing rewards).

Further, some techniques can be applied to some of the machine learning algorithms. For example, feature learning can be used. In other words, the machine learning model may be trained at least in part using feature learning, and/or the machine learning algorithm may include a feature learning component. Feature learning algorithms, called representation learning algorithms, may preserve the information in their input but transform it in such a way that it becomes useful, often as a pre-processing stage before performing classification or prediction. Feature learning can be based on principal component analysis or cluster analysis, for example.

In some examples, anomaly detection (i.e., outlier detection) may be used, which aims to provide identification of input values that raise suspicion because they differ significantly from the majority of input and training data. In other words, the machine learning model may be trained at least in part using anomaly detection, and/or the machine learning algorithm may include an anomaly detection component.

In some examples, the machine learning algorithm may use a decision tree as a predictive model. In other words, the machine learning model may be based on a decision tree. In a decision tree, observations about an item (e.g., a set of input values) can be represented by the branches of the decision tree, and an output value corresponding to the item can be represented by the leaves of the decision tree. Decision trees can support both discrete values and continuous values as output values. When discrete values are used, the decision tree can be called a classification tree; when continuous values are used, the decision tree can be called a regression tree.

Association rules are another technique that can be used in machine learning algorithms. In other words, the machine learning model can be based on one or more association rules. Association rules are created by identifying relationships between variables given large amounts of data. The machine learning algorithm may identify and/or use one or more ratio rules that represent knowledge that is inferred from the data. The rules may be used, for example, to store, manipulate, or apply the knowledge.

Machine learning algorithms are typically based on a machine learning model. In other words, the term "machine learning algorithm" may refer to a set of instructions that can be used to create, train, or use a machine learning model. The term "machine learning model" may refer to a data structure and/or set of rules representing the learned knowledge (e.g., based on training performed by the machine learning algorithm). In embodiments, the use of a machine learning algorithm may imply the use of an underlying machine learning model (or a plurality of underlying machine learning models). The use of a machine learning model may imply that the machine learning model and/or the data structure/set of rules that is/are the machine learning model is trained by a machine learning algorithm.

For example, the machine learning model may be an artificial neural network (ANN; artificial neural network). ANNs are systems inspired by biological neural networks, such as those found in a retina or brain. ANNs include a plurality of interconnected nodes and a plurality of connections, called edges, between nodes. There are typically three types of nodes, input nodes that receive input values, hidden nodes that are connected (only) to other nodes, and output nodes that provide output values. Each node can represent an artificial neuron. Each edge can send information, from one node to another. The output of a node can be defined as a (nonlinear) function of its inputs (e.g., the sum of its inputs). The inputs of a node may be used in the function based on a "weight" of the edge or node providing the input. The weight of nodes and/or of edges can be adjusted in the learning process. In other words, training an artificial neural network may include adjusting the weights of the nodes and/or edges of the artificial neural network, i.e., to achieve a desired output for a given input.

Alternatively, the machine learning model may be a support vector machine, a random forest model, or a gradient boosting model. Support Vector Machines (i.e., support vector networks) are supervised learning models with associated learning algorithms that can be used to analyze data (e.g., in a classification or regression analysis). Support Vector Machines can be trained by providing an input with a plurality of training input values belonging to one of two categories. The Support Vector Machine can be trained to assign a new input value to one of the two categories. Alternatively, the machine learning model may be a Bayesian network, which is a probabilistic directed acyclic graphical model. A Bayesian network may represent a set of random variables and their conditional dependencies using a directed acyclic graph. Alternatively, the machine learning model can be based on a genetic algorithm, which is a search algorithm and heuristic technique that mimics the process of natural selection.

## Claims

1. A computer-implemented method for predictive assessment of a health risk parameter, such as a risk for diabetes, in particular gestational diabetes mellitus, and/or pre-eclampsia, for a woman based on at least one pregnancy-related parameter, comprising the following steps:
providing an assessment of the health risk parameter using a first machine-learning model (1) if the pregnancy-related parameter fulfils a first condition;
providing an assessment of the health risk parameter using a second machine-learning model (2) if the pregnancy-related parameter fulfils a second condition; and
making available the health risk parameter.

2. The method of claim 1, wherein the first and second condition are of such nature that they mutually exclude each other.

3. The method of claim 1 or 2, wherein the pregnancy-related parameter comprises a number of pregnancies already carried out by the woman and/or an ordinal number count of an ongoing pregnancy.

4. The method of any of the preceding claims, wherein the first condition is fulfilled if the woman is having her first pregnancy and/or wherein the second condition is fulfilled if the woman is having her second or subsequent pregnancy.

5. The method of any of the preceding claims, wherein the first machine-learning model (1) and the second machine-learning model (2) are deployed on separate cohorts.

6. The method of claim 5, wherein the first machine-learning model (1) and the second machine-learning model (2) have been trained on a combined cohort.

7. The method of claim 5, wherein the first machine-learning model (1) and the second machine-learning model (2) have been trained on separate cohorts.

8. The method of any of the preceding claims, further comprising:
providing an assessment of the health risk parameter using a third machine-learning model (3) if the pregnancy-related parameter fulfils the second condition as well as a third condition, in particular a third condition relating to a prior history of the woman with respect to the health condition whose risk is assessed using the health risk parameter.

9. The method of claim 8, further comprising:
providing an assessment of the health risk parameter using the second machine-learning model (2) if the pregnancy-related parameter fulfils the second condition, but not the third condition, in particular with the third condition relating to a prior history of the woman with respect to the health condition whose risk is assessed using the health risk parameter.

10. The method of any of the preceding claims, performed during the first trimester of an ongoing pregnancy.

11. A computer-implemented machine-learning model structure for predictive assessment of a health risk parameter, such as a risk for diabetes, in particular gestational diabetes mellitus, and/or pre-eclampsia, for a woman based on at least one pregnancy-related parameter, comprising a first machine-learning model (1) and a second first machine-learning model (2), wherein
the first machine-learning model (1) may provide an assessment of the health risk parameter if the pregnancy-related parameter fulfils a first condition; and
the second machine-learning model (2) may provide an assessment of the health risk parameter if the pregnancy-related parameter fulfils a second condition.

12. A method of training the machine-learning model structure of claim 11, comprising
training one or more machine-learning models using nulliparous and multiparous cohort data; and
deploying the one or more machine-learning models on separate cohorts as first machine-learning model and the second machine-learning model;
particularly:
wherein training the first machine-learning model (1) is based on the nulliparous cohort data whereas training the second machine-learning model (2) is based on the multiparous cohort data.

13. A data processing device such as a computer or a mobile phone, in particular an electronic personal and/or professional health device, or a controller for such a data processing device, comprising means for carrying out the method of any one of the preceding claims 1-10 or 12.

14. A computer program, or a computer-readable medium storing a computer program, the computer program comprising instructions which, when the program is executed on a computer, cause the computer to carry out the method of any one of claims 1-10 or 12.

15. A data structure comprising the machine-learning model structure of claim 11.
